# EUROPEAN PATENT SPECIFICATION

(11) **EP 3 378 042 B1**
(45) Date of publication and mention of the grant of the patent: **16.09.2020**
(21) Application number: 16805486.4
(22) Date of filing: 17.11.2016
(51) Int. Cl.: G06T 11/00

(54) **METHODS AND APPARATUSES FOR PENETRATING IMAGING**
VERFAHREN UND VORRICHTUNGEN ZUR DURCHDRINGUNGSBILDGEBUNG
PROCÉDÉS ET APPAREILS D'IMAGERIE DE PÉNÉTRATION

(30) Priority: 18.11.2015 FI 20155856
(43) Date of publication of application: 26.09.2018
(73) Proprietor: Teknologian tutkimuskeskus VTT Oy, 02150 Espoo (FI)
(72) Inventor: LÖTJÖNEN, Jyrki, 33101 Tampere (FI); KORTELAINEN, Juha M., 33101 Tampere (FI)
(74) Representative: Kolster Oy Ab
(86) International application number: PCT/FI2016/050809
(87) International publication number: WO 2017/085360

(56) References cited:
- WO-A1-2015/044837
- ISOLA A A ET AL: "Motion-compensated iterative cone-beam CT image reconstruction with adapted blobs as basis functions; Motion-compensated iterative cone-beam CT reconstruction with blobs", PHYSICS IN MEDICINE AND BIOLOGY, INSTITUTE OF PHYSICS PUBLISHING, BRISTOL GB, vol. 53, no. 23, 7 December 2008 (2008-12-07), pages 6777-6797, XP020141531, ISSN: 0031-9155, DOI: 10.1088/0031-9155/53/23/009

## Description

### FIELD OF THE INVENTION

The present invention relates to penetrating imaging of a three-dimensional ("3D") object, which in a typical but non-restrictive use case is a patient. A typical but non-restrictive example of penetrating imaging is cone beam computed tomography ("CBCT").

### BACKGROUND OF THE INVENTION

Much of the present disclosure applies for use cases wherein the scanned 3D object is a patient, from whom anatomic data is obtained for diagnostic and/or therapeutic purposes by means of CBCT imaging. It should be remembered, however, that the present disclosure is equally applicable to use cases wherein the scanned 3D object is not a patient, such as an organism or inanimate object for various purposes, including research, examination, quality control, fault detection, or the like. It should be understood that the word "cone" is frequently used in a loose sense, to cover any implementations wherein a point source outputs penetrating radiation in multiple directions. For instance, the multiple directions may take the shape of a fan or pyramid, for example. Furthermore, the penetrating radiation is not necessarily restricted to X-ray radiation, and the present disclosure is also applicable to other forms of penetrating imaging, such as ground-penetrating radar imaging or foliage-penetrating laser imaging, to name but a few exemplary techniques.

Still using CBCT as a non-restrictive example, CBCT has become increasingly important in treatment planning and diagnosis in various fields, including but not limited to implant dentistry and interventional radiology (IR). In the exemplary field of dentistry, CBCT scanners are now finding many uses, such as in the fields of oral surgery, endodontics and orthodontics. Integrated CBCT is also an important tool for patient positioning and verification in image-guided radiation therapy (IGRT).

During dental imaging, the CBCT scanner rotates around the patient's head, obtaining a sequence of distinct two-dimensional ("2D") images. The CBCT scanner typically rotates by approximately half a degree between two consecutive scans and the resulting sequence typically comprises hundreds of images, such as about 600 images. For Interventional Radiology, the patient may be positioned offset to the table so that the region of interest ("ROI") is centered in the field of view for the cone beam, and a single rotation, typically spanning about 200 degrees, over the ROI acquires a volumetric data set. The volumetric data set, ie, the sequence of 2D images, is processed by an on-board or external scanning software, which acquires the data and reconstructs it, producing what is termed a digital volume composed of 3D volumetric picture elements ("voxels") of anatomical data that can then be manipulated and visualized with specialized software.

Acquisition of the hundreds of 2D images typically requires up to 40 seconds. During the image-acquisition process, the patient may move. The patient's movement during the image-acquisition process may cause significant blurring in the reconstructed volumetric image. Rescanning is avoided, if possible, to minimize X-ray exposure. Restricting the patient's movements is often impractical, impossible, uncomfortable and/or time-consuming.

Document WO 2015/044837 A1 discloses a motion compensated iterative reconstruction wherein the estimated motion is represented as a pre-computed motion vector field describing the movement of each image voxel between different motion phases.

US Patent Application 2011/0176723 by Imad Ali et al. discloses techniques in which internal or external markers are affixed to the patient. The markers typically contain metal, which strongly blocks X-rays. As a result, the positions of the markers can be tracked over the sequence of images. Internal markers are implanted within the patient and external markers are affixed to the patient's skin.

The Ali et al. disclosure suffers from certain residual problems. For instance, affixing the markers is a time-consuming operation, the patient may find the markers uncomfortable and the markers, while helping reduce motion artefacts, may cause imaging artefacts of their own.

### SUMMARY OF THE INVENTION

It is an object of the present invention to provide methods, equipment and computer program products, which alleviate one or more of the problems identified above.

An aspect of the present invention is a method according to claim 1.

Step a) is known from the prior art. Herein, "penetrating imaging apparatus" means an apparatus that forms an image of a three-dimensional ("3D") object via radiation originating from a point source into multiple directions, penetrating the 3D object and captured by a detector. A CBCT scanner is a typical but non-restrictive embodiment of a penetrating imaging apparatus. The orientation of the 3D object with respect to the imaging apparatus is incrementally altered, and a new image is captured. The orientation of the 3D object with respect to the imaging apparatus may be altered by rotating the 3D object within a stationary imaging apparatus, or the radiation source and detector may be rotated around the 3D object, which remains stationary. "Stationary" is an ideal objective which is virtually never attained in when the 3D object is a living organism. Instead, the 3D object experiences an unknown transformation during the scanning period. It should be understood that the unknown transformation can be highly complex. Assuming that the 3D object is modeled via finite elements, each individual element may be transformed separately between two consecutive images. One way to model such transformations is combining shifts along three coordinate axes plus rotations about those axes. In embodiments wherein the 3D object is rotated deliberately within a stationary imaging apparatus, the deliberate rotation is "known transformation", from which the real 3D object deviates by the unknown transformation. The unknown additional transformation may be zero or close to zero but one of the problems to be solved by the present disclosure is that the data processing apparatus does not know *a priori* what the unknown additional transformation is.

Step b) is also known from the prior art. It comprises creating an initial reconstructed volumetric image by applying an initial reconstruction on the sequence of the images captured in step a).

Steps c) through e) constitute an image-correction process by which the effects of the unknown transformations will be reduced. The image-correction process comprises creation of varied spatial transformation parameters for each of multiple imaging angles, each of which corresponds to the image captured at that imaging angle. For each imaging angle, multiple sets of spatial transformation parameters are created.

In this disclosure the term "spatial transformation" refers to terminology used in image registration. In image registration, two objects are aligned with each other by transforming one object to the other one using a spatial transformation. In the simplest form, one object is translated (shifted) to match with the other object. When the transformation combines translation and rotation, it is called a rigid-body transformation. Scaling and skewing of objects belong to the group of affine transformations incorporating non-rigid components into the transformation. Degrees of freedom can be further added. Finally a translation vector can be attached to each point of the object. For example, if a volumetric image consists 256x256x256 voxels, the non-rigid transformation contains 3x256x256x256 parameters (degrees of freedom), "3" coming from a translation vector for x-, y- and z-axes. This disclosure relates to registering a reconstructed volumetric image (3D) to captured images (2D) by finding spatial transformation(s) which reconstruct the unknown transformation(s) that occurred during the imaging.

In a basic implementation, the different spatial transformation parameters may only contain shifts along the coordinate axes. Or, in view of the fact that shifts along the imaging angle do not cause significant artefacts, the different spatial transformation parameters may only contain shifts in the imaging plane, ie, the plane normal to the imaging angle. In more ambitious implementations, the different spatial transformation parameters may contain various combinations of shifts, rotations, scaling and/or skewing, possibly for various portions of the 3D object (and the reconstructed volumetric image, which models the 3D object) or even fully non-rigid transformations of the 3D object represented by a transformation vector at each location of the object. For instance, the spatial transformation parameters may be different for a patient's jaw compared with the rest of the head, or fully non-rigid for the patient's thorax due to breathing. Applying different transformation parameters can be implemented in multiple ways. Either the reconstructed volume (step b or step e) can be altered by transformations, or different factors related to the imaging device can be altered, for instance by varying the locations and/or orientations of the source and detector of the imaging device but keeping the reconstructed volume unaltered, or altering both the reconstructed volume and the factors of the imaging device.

For each of several imaging angles, a set of simulated images is created, for instance by projecting virtual rays through the initial reconstructed volumetric image from the source to the detector, under a set of varied spatial transformation parameters of the initial reconstructed volumetric image or a portion of it, relative to the imaging apparatus, wherein the simulated images within the set differ from one another by the set of varied different spatial transformation parameters of at least a portion of the initial reconstructed volumetric image. Such projecting of virtual rays simulates the real imaging of step a) by replacing the real 3D object by the initial reconstructed volumetric image. Those skilled in the art will understand that "projecting" does not require a physical projector. In the present context, projecting can be implemented by simulating propagation of energy (eg light, X-ray, ultrasound or the like) from a multidimensional source image to a target image having fewer dimensions than the source image. In typical implementations the source image for the projection is the initial reconstructed volumetric image in a first (and possibly the only) execution of the transformation-correction process, and in subsequent, iterative, executions the source image may be the transformation-corrected reconstructed volumetric image resulting from the previous execution of the process.

Next, within each set of the simulated images, a set of optimized set of spatial transformation parameters is determined. Let us first assume that a search for "optimal" spatial transformation parameters is carried out. In this task, an image most similar with the captured image associated with the common imaging angle of the set is searched. By determining the most similar, ie, best-fitting, simulated image for each imaging angle, the data processing apparatus also knows which of the different spatial transformation parameters resulted in the best-fitting images. The process of generating simulated images and finding the most similar image can be implemented in multiple ways. For instance, the process may involve generating a large number of simulated images in the beginning, followed by searching for the best-fitting simulated image, which also yields the spatial transformation parameters under which the best-fitting simulated image was created. As another example, the search for "optimal" spatial transformation parameters may be implemented iteratively using different optimization techniques, such as gradient optimization, simplex method, genetic algorithms or simulated annealing.

In real-world implementations it may not be cost-effective to search for the truly "optimal" image and the criterion can be relaxed from "optimal" to "optimized". In the present context, the search for an optimized set of spatial transformation parameters means determination of a set of spatial transformation parameters which, when used for the simulated projection, results in an improved fit with the captured image associated with the imaging angle (φ). Herein, an improved fit means a fit better than the fit of the simulated projection in absence of the spatial transformation parameters. Thus an "optimized" set refers to a set of spatial transformation parameters which has undergone an optimization process which may or may not yield absolutely optimal values. For the purposes of the present disclosure, what matters is that the optimized spatial transformation parameters provide some improvement compared with absence of the parameters. Relaxing the criterion from optimal to optimized is likely to reduce processing time, which may result in improved throughput. Alternatively, the reduced processing time may be utilized by performing the image-correction process in multiple iterations, substituting the transformation-corrected reconstructed volumetric image from iteration i for the initial reconstructed volumetric image in iteration i+1.

In step e), the image reconstruction of step b is repeated, except that the set of optimized spatial transformation parameters determined in step d) for each imaging angle is taken into account when producing the second reconstruction. It is not necessary that steps b) and e) use the same reconstruction algorithm and its parameters, and those can be changed also between different iterations at step e. For instance, using less accurate but faster reconstructions in the beginning can be computationally beneficial. The result of step e) is a transformation-corrected reconstructed volumetric image. In some embodiments, the set of optimized spatial transformation parameters determined in step d) is taken into account computationally in the reconstruction algorithm by updating the factors of the imaging device to compensate for movements of the 3D object and/or non-idealities of the imaging geometry. In other embodiments, the original captured images can be transformed based on the set of optimized transformation parameters determined in step d). In still other embodiments, both previously described techniques can be combined.

Another aspect of the invention is a programmed data processing apparatus specifically adapted to carry out the method described above. Yet another aspect of the invention is a computer program product whose execution in a data processing apparatus causes execution of the method described above.

The above-described method steps, or the corresponding program instructions for the data processing apparatus, may be further modified by various optional features. For instance, in some embodiments the above-described method may be repeated iteratively, as stated above.

Furthermore, it should be noted that step c) is formulated in such a manner that the multiple different spatial transformation parameters for each imaging angle can be generated prior to applying the simulated projections, which are varied by the different spatial transformation parameters, or the different spatial transformation parameters may be created "on the fly", as the simulated projections are being generated. In some implementations it is beneficial to create all different spatial transformation parameters before applying them in the simulated projection phase.

In some embodiments, the spatial transformation parameters are generated for at least one portion of the 3D object (and the volumetric image) separately from the remainder of the 3D object. This makes it possible to model transformations for portions of the 3D object, such as members or organs of an organism, separately. For instance, the separately modeled portions may include the jaw, heart or lungs of a patient, or rigid portions of inanimate objects coupled by flexible joints.

In some embodiments the act of determining the optimized set of spatial transformation parameters comprises filtering the spatial transformation parameters in one or more combinations, wherein each combination comprises spatial transformation parameters for multiple imaging angles. The filtering process aims at increasing continuity of spatial transformation parameters across different imaging angles by processing the spatial transformation parameters from more than one imaging angle in one or more combinations. Filtering the spatial transformation parameters in combination(s) may be accomplished in a number of ways, one of which comprises generating a multi-dimensional path or "trajectory" of the combined spatial transformations and then smoothing the trajectory, possibly in several segments. For instance, running median smoothing has been found to remove outliers effectively.

When the different spatial transformation parameters for each imaging angle are generated, it is beneficial to omit shifts in the direction of the imaging angle. This simplification reduces computational load and may contribute to increased robustness. The latter benefit is achieved because shifts in the direction of the imaging axis have negligible contribution to imaging artefacts or to corrections thereof, it is best to omit corrections in the direction in which the corrections are ineffective.

In some implementations, robustness is further improved by omitting the captured image associated with imaging angle φ when the set of shifted reconstructed volumetric images associated with imaging angle φ is generated.

### BRIEF DESCRIPTION OF THE DRAWINGS

In the following section, specific embodiments of the invention will be described in greater detail in connection with illustrative but non-restrictive examples. A reference is made to the following drawings:
Figure 1 shows an illustrative setup for a CBCT scanner with a 3D object, which typically is a patient;
Figure 2 is a flow chart illustrating processing phases in an embodiment of the invention;
Figures 3A and 3B, which form a single logical drawing, illustrate various processing phases of an embodiment of the invention in the form of data structures;
Figure 4 shows an exemplary data processing architecture adapted to perform the various data processing tasks relating to embodiments of the invention; and
Figure 5 illustrates results obtained from various experiments.

### DETAILED DESCRIPTION OF SOME SPECIFIC EMBODIMENTS

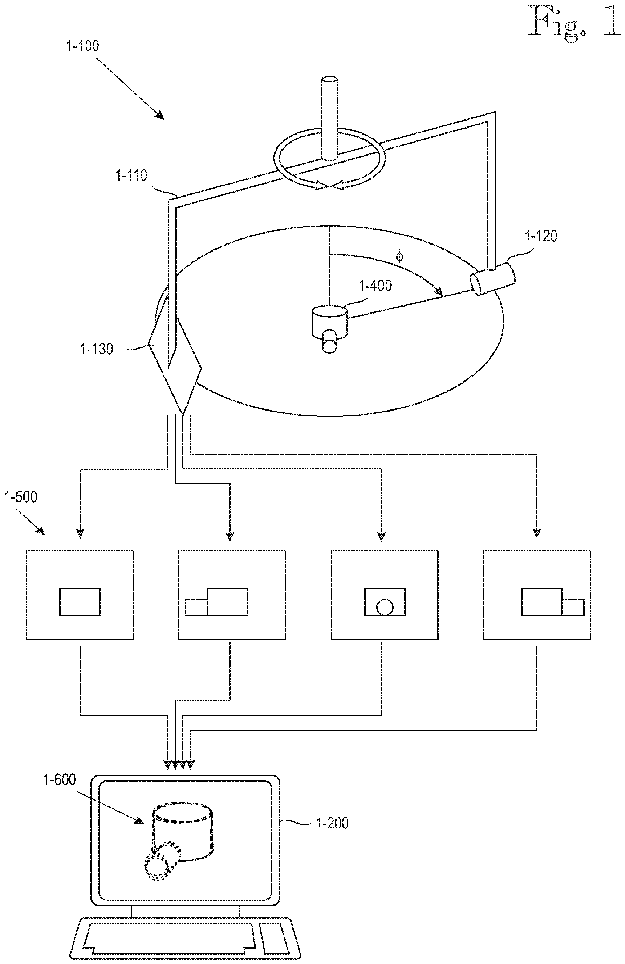

Figure 1 shows an illustrative setup for a penetrating imaging apparatus with a 3D object, which typically is a patient. The present disclosure uses two-part reference numbers wherein the first digit indicates the drawing figure in which the item is first introduced. The penetrating imaging apparatus is assumed to be a CBCT scanner, which is a typical but non-restrictive embodiment.

The CBCT scanner is generally denoted by reference number 1-100. It typically comprises a supporting structure 1-110, known as a gantry. The gantry 1-110 supports an X-ray source 1-120. The X-ray source typically emits a conical beam of X-rays towards a 3D object being scanned, although other shapes for the beam are possible, such as a pyramid or fan. The 3D object, which typically is a patient or a portion of a patient, is denoted by reference number 1-400. The X-rays traverse the "patient" (3D object) 1-400 and hit an X-ray detector 1-130. While the X-rays traverse the patient 1-400, on their way from the source 1-120 to the detector 1-130, they are absorbed to a varying degree, depending on the cumulative absorption of the tissue (or structure) of the patient that each ray has to traverse.

To keep the description simple, all image processing is assumed to take place in one data processing apparatus ("computer"), denoted by reference number 1-200. In actual implementations, it may be practical to distribute image processing among multiple computers, each of which performs a subset of the image-processing tasks.

In the illustrated implementation, while the gantry 1-110 rotates the X-ray source 1-120 and detector 1-130 around the patient 1-400 over a proscribed arc, output signals from the detector 130 are provided to the computer 1-200, which processes them as a sequence of 2D captured images, generally denoted by reference number 1-500. While the "patient" 1-400 is three-dimensional, each of the captured images 1-500 is two-dimensional. For each 2D captured image, its imaging angle φ is determined from the mutual orientations of the X-ray source 1-120 and detector 1-130 with respect to the patient 1-400 at the time when the 2D captured image was captured.

Those skilled in the art will understand that Figure 1 does not accurately illustrate the CBCT imaging process because a strict interpretation of patenting regulations prohibits grayscale images. More realistic images will be presented later in this disclosure, under the heading "Experiments".

It is customary but not necessary to model the 3D object or patient by finite elements, while the corresponding elements in the volumetric image are called voxels ("volumetric pixels"). While an X-ray traverses the patient 1-400 on its way from the source 1-120 to the detector 1-130, the X-ray is attenuated by the cumulative absorption of all elements of the patient traversed by the X-ray. Accordingly, each pixel of one of the 2D captured images 1-500 indicates the cumulative absorption of all elements traversed the X-ray when fired at the specific direction, which is determined by the mutual orientation of the source 1-120, the patient 1-400 and the relative position of the respective photosite of the detector, which outputs the intensity value of the pixel. From the intensity value of the pixel of a single 2D captured image it is impossible to determine individual contributions of the traversed elements to the total absorption experienced by the X-ray that resulted in the given intensity value.

A known CBCT scanner (or the computer coupled to the scanner) computes a voxel-by-voxel combination of the 2D captured images 1-500 and thus generates a reconstructed volumetric image. In the present illustration, the reconstructed volumetric image can be displayed by the computer as volumetric presentation (3D view), denoted by reference number 1-600. A problem with the known CBCT scanner is that unknown transformations (shifts and rotations) of the patient 1-400 with respect to the X-ray source 1-120 cause various image artefacts, such as general blurring, halos or aberrations. Motion of the X-ray source 1-120 around the patient 1-400 is ideal if it adheres to a mathematically perfect trajectory and the patient is rigid and motionless. Conversely, non-idealities are caused by spatial transformations of the patient as a whole or portions of the patient, any mechanical play in the gantry, vibration of the patient's support, errors of various position and rotation sensors in the gantry, or the like. An object of the present disclosure is to alleviate the imaging artefacts caused by one or more of the patient's spatial transformations during scanning, non-ideal motion of the components of the scanner, and other related non-idealities, and to avoid the additional problems caused by the internal and external metal markers used by Ali et al.

Instead of modeling the 3D object or patient by finite discrete elements, and the volumetric image by discrete voxels, such modeling can be based on continuous mathematical functions, such as spherical harmonics functions.

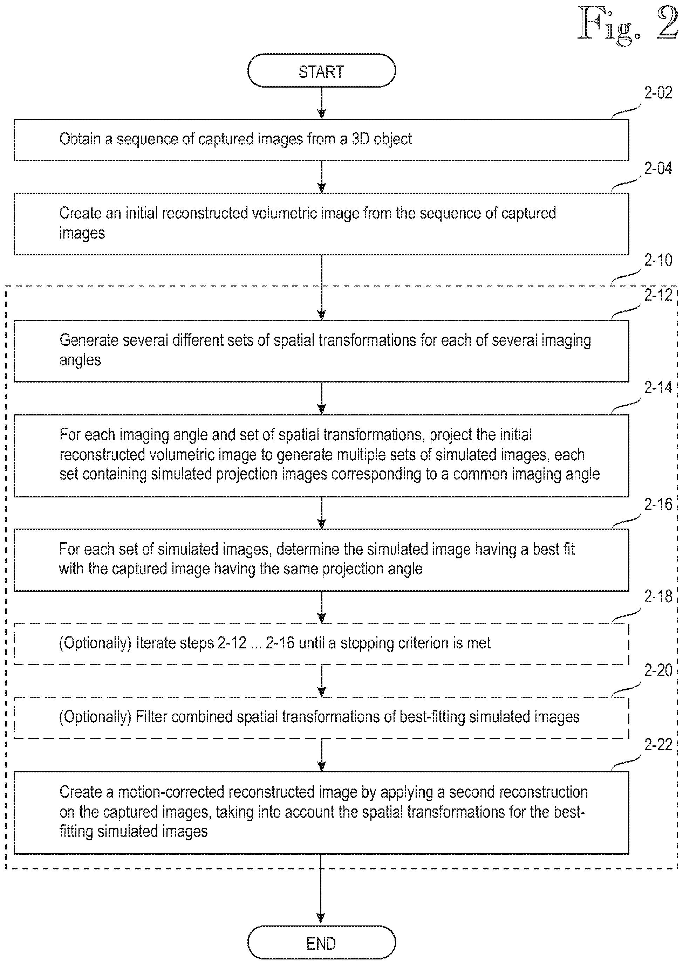

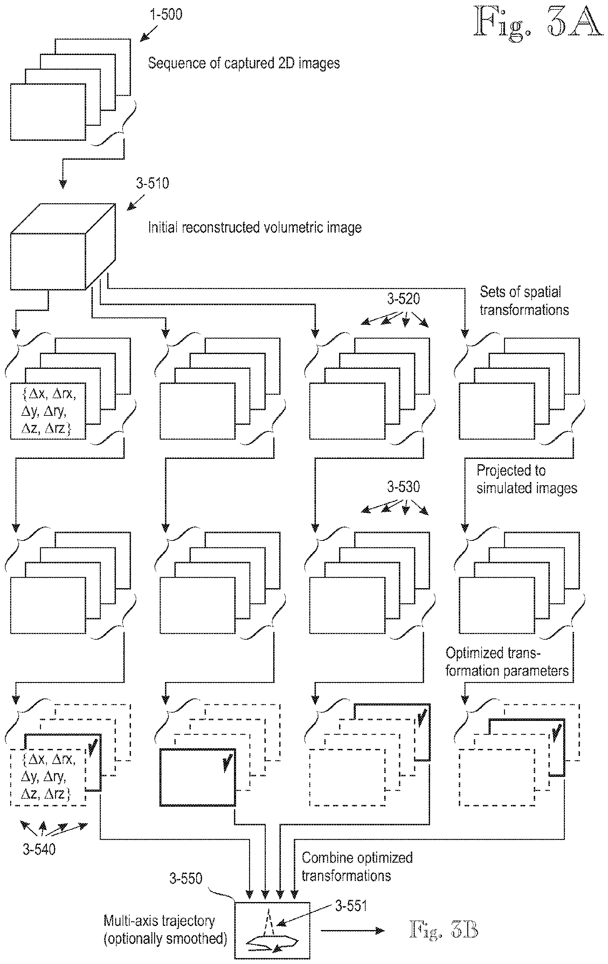

Various embodiments for the processes by which the above objects can be attained will be described in connection with Figures 2 and 3A - 3B, of which Figure 2 shows processing phases and Figures 3A - 3B show various related data structures in an embodiment of the invention.

In step 2-02, the data processing apparatus (schematically shown as a singular computer 1-200 in the embodiment of Figure 1) obtains a sequence of two-dimensional captured images 1-500. This step is well known in the prior art. Depending on implementation, the computer 1-200 may control the CBCT scanner 1-100 and obtain the captured images as they are captured by the sensor 1-130 while it rotates around the patient 1-400, or the computer 1-200 may be separate from the one that controls the imaging process, in which case the computer 1-200 may receive the sequence of captured images 1-500 from the separate image-acquisition processor internal to the CBCT scanner 1-100. Each captured image in the sequence 1-500 is associated with an imaging angle, which depends on the mutual orientations of the X-ray source 1-120 and detector 1-130 with respect to the patient 1-400 at capture time.

In step 2-04 the computer 1-200 uses the sequence of 2D captured images 1-500 to create an initial reconstructed volumetric (3D) image 3-510. The reconstructed volumetric (3D) image may be based on a discrete voxels or continuous functions, such as spherical harmonics functions. Creation of the initial reconstructed volumetric image 3-510 is also known from the prior art. Those skilled in the art will realize that each of the captured 2D images 1-500 is a view of the patient from a single imaging angle, while the reconstructed volumetric image 3-510 is a 3D image, from which visualizations can be projected in any imaging angle.

As will be evident from the following description, the present disclosure deviates from the teaching of Ali et al. in that Ali teaches (eg in Figure 2) to perform all image-correction steps 204 - 214 before the 3D reconstruction step 216. A computer according to the present disclosure cannot rely on easily identifiable metal markers, which serve to indicate motion that requires correction. Instead the present disclosure teaches performing the initial 3D reconstruction 3-510 early, in step 2-04, and the initial reconstruction 3-510 provides a source for later image-correction steps.

Reference number 2-10 generally denotes an artefact reduction process by which the present invention is able to reduce motion-related imaging artefacts without additional metal markers. The computer of the present disclosure determines an optimized set of spatial transformation parameters for each imaging angle φ and then performs a second reconstruction of the captured images, taking into account the determined optimized spatial transformation parameters for each imaging angle φ. It should be noted, however, that there are various embodiments for the artefact reduction process and Figure 2 shows but one of them.

In the implementation shown in Figure 2, the computer, in step 2-12, first generates multiple sets of spatial transformation parameters 3-520 relative to the imaging apparatus, such as shifts, rotations, skews, magnifications or combinations of these. In step 2-14, the computer generates simulated projection images 3-530, all corresponding to the imaging angle φ, by repeatedly applying simulated projections 2-14 on the initial reconstructed volumetric image under a respective set of spatial transformation parameters 3-520. Instead of applying the spatial transformation parameters on the entire reconstructed volumetric image, the computer may apply the spatial transformation parameters on a portion of the reconstructed volumetric image and thereby attempt to correct for imaging artefacts for that portion separately. A non-restrictive example is processing a dental patient's chin separately from the rest of the head.

In step 2-16, the computer determines an optimized set of spatial transformation parameters 3-540 within the set of the simulated images 3-530 corresponding to the imaging angle φ. Again, there are several possible implementations for this step. In the simple illustration shown in Figure 2, the computer first, in steps 2-12 and 2-14, generates large numbers of spatial transformation parameter sets 3-520, and uses them to generate differently transformed simulated projections 3-530. Then the computer, in step 2-16, selects the transformed simulated projection for imaging angle φ, which yields the best fit (or minimizes an error function) with the captured image for the same imaging angle φ.

A residual problem with the implementation shown as steps 2-12 ... 2-16 is that generating all candidate spatial transformations and the corresponding transformed simulated projections may require huge amounts of storage. For instance, ten values for each of six transformations (3D shifts and rotations) results in 10⁶ simulated projections for each imaging angle. This is one of the reasons for generally avoiding the term "optimal transformation parameters" in this disclosure: there may not be enough time to determine the absolutely optimal transformation parameters. Instead, the present disclosure teaches determining an optimized set of spatial transformation parameters which, when used for the simulated projection, results in an improved fit with the captured image associated with the imaging angle. Improved fit means a fit having a higher similarity value (eg normalized cross correlation) or lower error value with the captured image for imaging angle φ than does the simulated projection without application of the spatial transformation parameters. Reference number 3-540 denotes the optimized sets of spatial transformation parameters for respective imaging angles φ.

Instead of generating all spatial transformations and corresponding simulated projections, or a large number of them, in one go, the search for an optimized set of spatial transformation parameters, namely steps 2-12 ... 2-16 may be performed iteratively. Reference number 2-18 schematically denotes such an iteration process. In one non-restrictive example of iteration, the computer generates in step 2-12 one set of spatial transformation parameters 3-520 for each parameter, such as shifts and rotations about three axes. Step 2-14 involves generating a respective simulated projection 3-530 for each of the sets 3-520, while step 2-16 involves determining the set which results in the best fit with the captured image corresponding to the same imaging angle. In the iterative implementation, step 2-18 comprises returning to step 2-12 until a stopping criterion is met. For instance, the stopping criterion may be met if further changes to the transformations fail to yield fit improvements over some threshold value, or if the iterative process 2-18 has consumed all the time allocated to it.

In an optional step 2-20 the computer filters the optimized sets of spatial transformation parameters 3-540 in multiple dimensions, for example by combining the spatial transformation parameters into an initial multi-dimensional trajectory 3-550, which indicates transformations for each of the best-fitting simulated images. The multi-dimensional trajectory 3-550 also is a motion estimate for modeling the unknown movement of the patient and/or non-idealities of the motion of the CBCT scanner during the scanning process. The multi-axis trajectory 3-550 is then subjected to a filtering or refining process, for example by smoothing. The inventors have experimented with various smoothing techniques and found running median smoothing to be a particularly effective technique, possibly because it removes outliers effectively. In Figure 3A, reference number 3-551 denotes an outlier, which has been removed in the smoothing step 2-20. Those skilled in the art will realize that it is impossible to give an accurate 2D presentation of a trajectory which describes transformations in more than two dimensions, but the iconic presentation 3-551 symbolizes the fact that outliers may be removed from a combined trajectory before using any of the transformations to create the reconstructed volumetric image.

After the optional trajectory-creation and smoothing step 2-20, the computer performs step 2-22, which comprises applying a second reconstruction on the sequence of captured images. The second reconstruction 2-22 is basically analogous with the initial reconstruction 2-04 except that the second reconstruction 2-22 takes into account the optimized set of spatial transformation parameters determined for each imaging angle φ. For instance, shifts perpendicular to the imaging axis can be realized by slightly altering the imaging angle φ when the captured image for that imaging angle is utilized in the reconstruction. Step 2-22 thus results in a transformation-corrected reconstructed volumetric image, denoted by reference number 3-560.

This completes the description of a basic implementation of a method according to the present disclosure. The description continues with additional optional features, which aim at solving residual problems and/or providing additional benefits.

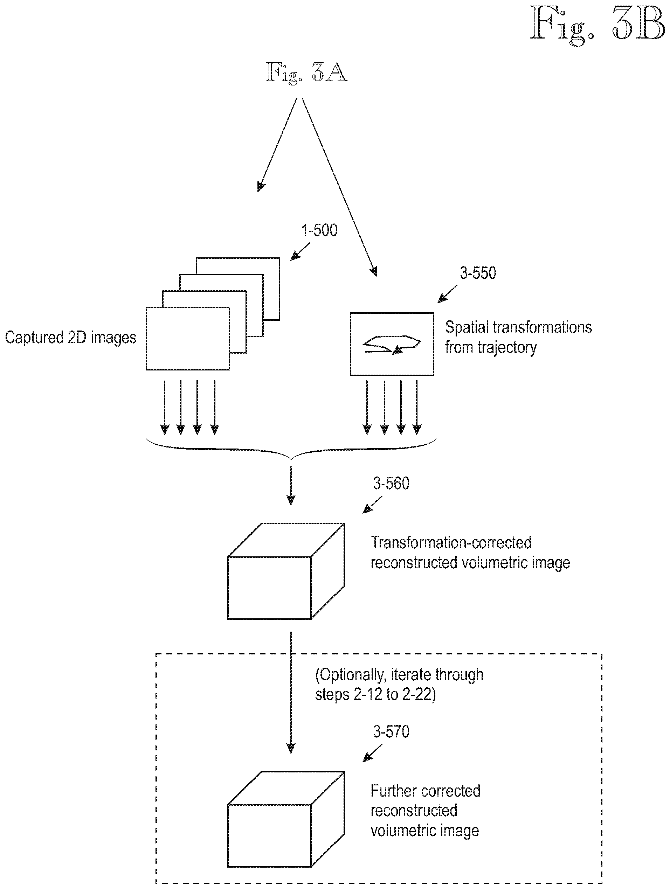

According to one optional feature, the entire process shown and described in connection with Figure 2, apart from the image-capturing step 2-02, is repeated iteratively. In one implementation of an iterative process, the transformation-corrected reconstructed volumetric image 3-560 is substituted for the initial reconstructed volumetric image 3-510 in step 2-04. In such an iterative implementation, the second reconstruction 2-22 is performed once for each iteration. In Figure 3B, reference number 3-570 denotes the result of such iterative performance of the process.

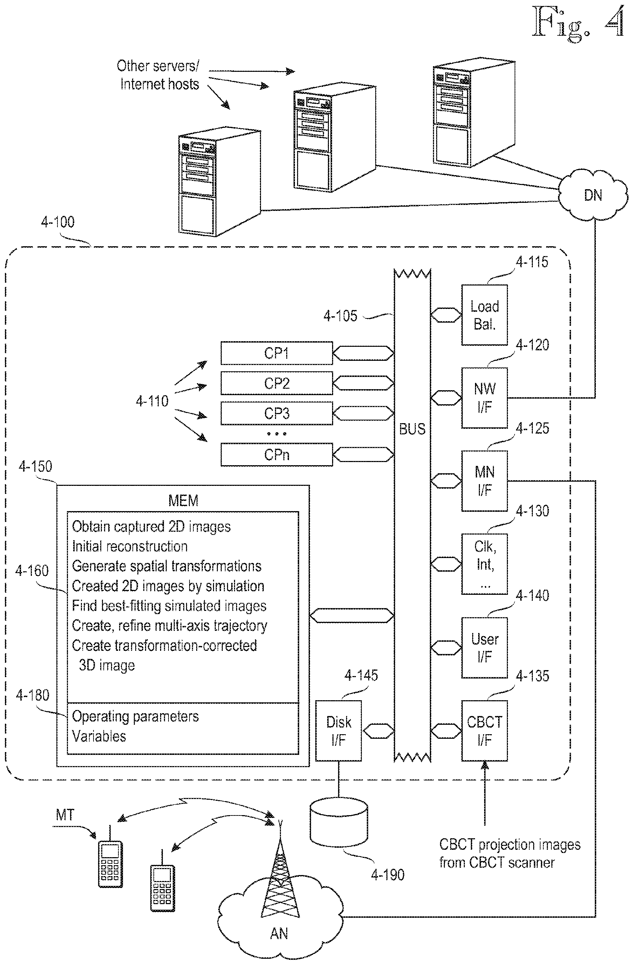

Figure 4 shows an exemplary data processing architecture adapted to perform the various data processing tasks relating to embodiments of the invention. In the following the data processing architecture will be referred to as a computer, but those skilled in the art will realize that the data processing architecture need not be implemented as a dedicated computer. Instead, several embedded techniques are possible, as are techniques in which the inventive functionality is installed on a data processing system that exists for other purposes.

The architecture of the computer, generally denoted by reference numeral 4-100, comprises one or more central processing units CP1 ... CPn, generally denoted by reference numeral 4-110. Embodiments comprising multiple processing units 4-110 are preferably provided with a load balancing unit 4-115 that balances processing load among the multiple processing units 4-110. The multiple processing units 4-110 may be implemented as separate processor components or as physical processor cores or virtual processors within a single component case. In a typical implementation the computer architecture 4-100 comprises a network interface 4-120 for communicating with various data networks, which are generally denoted by reference sign DN. The data networks DN may include local-area networks, such as an Ethernet network, and/or wide-area networks, such as the internet. In some implementations the computer architecture may comprise a wireless network interface, generally denoted by reference numeral 4-125. By means of the wireless network interface, the computer 4-100 may communicate with various access networks AN, such as cellular networks or Wireless Local-Area Networks (WLAN). Other forms of wireless communications include short-range wireless techniques, such as Bluetooth and various "Bee" interfaces, such as ZigBee or its some of its proprietary implementations.

The computer architecture 4-100 may also comprise a local user interface 4-140. Depending on implementation, the user interface 4-140 may comprise local input-output circuitry for a local user interface, such as a keyboard, mouse and display (not shown). The computer architecture also comprises memory 4-150 for storing program instructions, operating parameters and variables. Reference numeral 4-160 denotes a program suite for the server computer 4-100.

The computer architecture 4-100 also comprises circuitry for various clocks, interrupts and the like, and these are generally depicted by reference numeral 4-130. The computer architecture 4-100 further comprises a storage interface 4-145 to a storage system 4-190. The storage system 4-190 comprises non-volatile storage, such as a magnetically, optically or magneto-optically rewritable disk and/or non-volatile semiconductor memory, commonly referred to as Solid State Drive (SSD) or Flash memory. When the server computer 4-100 is switched off, the storage system 4-190 may store the software that implements the processing functions, and on power-up, the software is read into semiconductor memory 4-150. The storage system 4-190 also retains operating data and variables over power-off periods. The various elements 4-110 through 4-150 intercommunicate via a bus 4-105, which carries address signals, data signals and control signals, as is well known to those skilled in the art.

Reference number 4-135 denotes a CBCT interface by which the computer 4-100 obtains data from the CBCT scanner 1-100. Naturally, if the computing functions of the present disclosure is integrated in a CBCT scanner, a separate CBCT interface is not needed. The separate CBCT interface 4-135 may also be superfluous if the computer 4-100 communicates with the CBCT scanner 1-100 via any of the general-purpose interfaces, such as the network interface 4-120 or the mobile network interface 4-125.

The inventive techniques may be implemented in the computer architecture 4-100 as follows. The program suite 4-160 comprises program code instructions for instructing the processor or set of processors 4-110 to execute the functions of the invention or its embodiments, including: Acquisition of captured images (2-02), initial reconstruction based on obtained captured images (2-04), generation of sets of spatial transformation parameters (2-12), generation of simulated projections under the spatial transformation parameters (2-14), determination of optimized transformation parameter sets (2-16), optionally iteration of three preceding steps (2-18), optionally smoothing or otherwise filtering or refining combined optimized transformations (2-20) and creation of a motion-corrected reconstructed image by a second reconstruction (2-22) of the captured images, wherein the captured image for imaging angle φ is transformed by the optimized spatial transformation parameters for that imaging angle.

In addition to instructions for carrying out a method according to the invention or its embodiments, the memory 4-160 stores instructions for carrying out normal system or operating system functions, such as resource allocation, inter-process communication, or the like.

### REDUCTION OF SEARCH SPACE FOR BEST-FITTING IMAGES

Creation of the multiple sets of spatial transformation parameters and utilizing them in the artefact-reduction process may require huge amounts of computational resources. For instance, if a maximum correctable shift is 20mm in either direction (a total of 40mm), and the step size of shift correction is 1mm, the number of spatial transformations for each imaging angle is 40 in each dimension (x, y, z). Using these numbers, a total of 40³ = 64000 spatially transformed versions is created by simulation for each original captured 2D image. Note that these numbers only concern shifts. Taking rotations into account doubles the number of degrees of freedom and raises the number of spatially transformed versions of the 2D images to the second power of the original (40⁶).

The search space can be significantly reduced by ignoring shifts in the direction of the imaging axis (or the X-ray). This is because imaging artefacts caused by patient movement in the direction of the imaging axis or X-ray are less severe than artefacts caused by movements perpendicular to the imaging axis. Correspondingly, because the imaging axis is generally perpendicular to the image plane, shifts in the direction of the imaging axis have a negligible effect, which is why the image-correction algorithm might attempt huge shifts in the direction of the imaging axis, and such huge shifts may cause artefacts in other processing phases, such as the optional smoothing step 2-22. Ignoring shifts in the direction of the imaging axis or X-ray is equivalent to saying that the sets of differently multi-axis shifted reconstructed volumetric images, which are generated in step 2-12, are only shifted in the plane of the captured images (but not along the axis that is perpendicular to the image plane).

### CORRECTING FOR NON-IDEALITIES OF THE IMAGING APPARATUS

Most the above disclosure has focused on correcting for movements of the object being scanned ("patient"). A side effect of the artefact-reduction process described herein is that it also corrects for non-idealities of the imaging apparatus itself. But in some implementations it may be beneficial to determine optimized spatial transformation parameters for the imaging apparatus, while imaging a static subject, and use the parameters known to be optimal or close to optimal to reduce apparatus-related imaging artefacts before reducing artefacts caused by subject motion.

For instance, assuming that the gantry of the CBCT always rotates in the same direction during scanning, much of the mechanical play in the members and joints is presumably repeatable, as are other deviations from idealized geometry. The repeatable elements due to the scanner itself can be at least partially compensated for by using a static 3D object for scanning and finding optimal transformations for the best-fitting reconstructed volumetric images. These transformations, when determined with a static 3D object, can be saved and reused for later scans. Alternatively or additionally, some equipments may be provided by sensors for measuring the true position and orientation of the source and detector, and these are taken into account as known transformations in the reconstruction.

### TECHNIQUES FOR INCREASING ROBUSTNESS OF THE IMAGE-CORRECTION PROCESS

As stated under the heading "Reduction of search space for best-fitting images", restricting generation of shifts to the detector/image plane only, greatly reduces processing load. It also increases robustness by eliminating the opportunity to try large shifts in the direction of the imaging axis, because shifts in that direction are inefficient for image correction but may cause other artefacts.

Another technique, which can be used for increasing robustness of the image-correction process relates to steps 2-12 and 2-14, which comprise generating simulated images 3-530 corresponding to the imaging angle (φ), by simulated projections (2-14) of the initial reconstructed volumetric image under varied spatial transformation parameters (3-520) of the initial reconstructed volumetric image. The inventors have experimented with various techniques for generating the sets 3-520, and found out via simulations that it is beneficial to omit the captured image associated with imaging angle φ, and optionally some of its neighbors, when the set of simulated images corresponding to imaging angle φ is generated. It is difficult to provide a scientifically solid explanation for this discovery, but repeated simulations indicate that it increases robustness. One plausible explanation is as follows. Assume that the patient moved significantly at the time corresponding to imaging angle φ. The image captured at that time reflects the significant motion. Omitting the contribution of the affected captured image, and some of its neighbors, to the differently shifted reconstructed volumetric images thus filters out the movement that resulted in image degradation. On the other hand, if the patient did not move at the time corresponding to imaging angle φ, omitting the contribution of the corresponding captured image does not serious harm because its effect can be regenerated from the remaining captured images. The hypothesis is thus that the negative effects of the captured image associated with imaging angle φ on the quality of the simulated (projected) captured image exceed the positive effects, whereby the overall effect of the omission is generally beneficial. As an alternative to omitting the captured image associated with imaging angle φ, and optionally some of its neighbors, it may be beneficial to omit one or more captured images if none of the simulated projection images has a good enough fit (eg normalized cross correlation above predetermined threshold) with the captured image.

### EXAMPLES

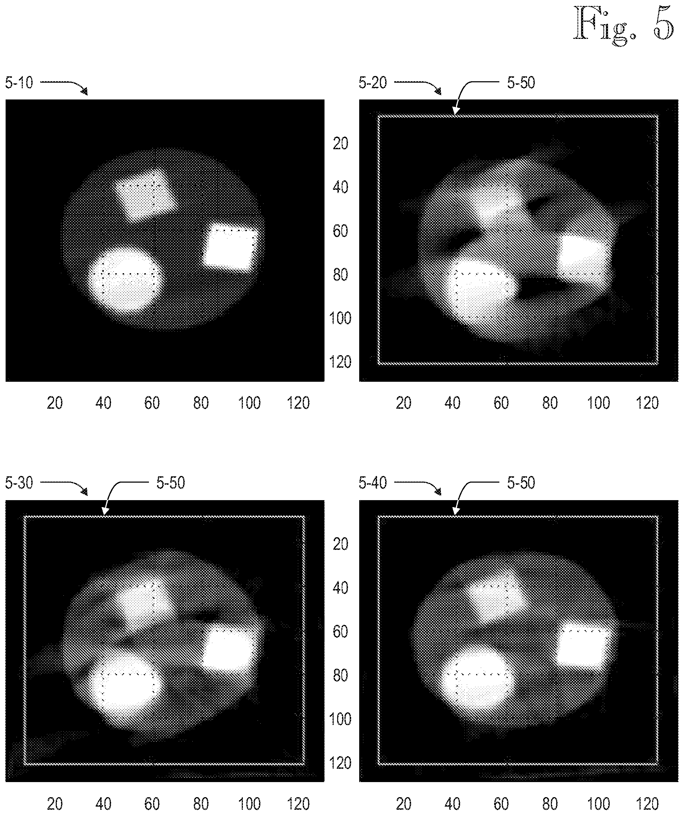

Figure 5 illustrates results obtained from various experiments. As stated above, deviations from idealized imaging geometry may have a variety of different causes, such as target movement during imaging, mechanical slack in the gantry, and so one. It is virtually impossible to accurately repeat such deviations from run to run, which is why all four images 5-10 through 5-40 in Figure 5 are simulated reconstruction results obtained in MatLab®. The simulations included ray-tracing through a virtual 3D "phantom" with and without target movement, with different embodiments of the present disclosure.

Reference numeral 5-10 denotes a simulation image of the 3D phantom without target movement. Therefore, as regards reduction of motion-related artefacts, the image 5-10 is an ideal reconstruction result because it was obtained without any target movement.

Reference numerals 5-20 through 5-40 denote simulation images obtained with similar target movement during the imaging process. Image 5-20 was obtained without any artefact reduction. Reference numeral 5-50 denotes an area over which a "fit" (similarity function) with the ideal reconstruction result 5-10 was calculated. In the examples shown herein, the fit was calculated as a normalized cross-correlation. The image 5-20 obtained without any artefact reduction has a fit of 0.86 with the ideal reconstruction result 5-10, as calculated over the area 5-50.

Reference numeral 5-30 denotes a simulation result obtained from a process as described in connection with Figure 2. The image 5-30 has a fit of 0.95 with the ideal reconstruction result 5-10, as calculated over the area 5-50. Compared with the fit 0.86 obtained under similar conditions (same target motion) but without motion artefact reduction, the improvement provided by the process of the present disclosure is significant. It should be noted, however, that the increase of the fit (cross-correlation) from 0.86 to 0.95 is a result attained in one experiment and does not restrict the scope of the process described herein.

Finally, reference numeral 5-40 denotes a simulation result obtained under similar conditions to the previous two images, when processed with a process as described in connection with Figure 2, which was further refined with the technique described in section "Techniques for increasing robustness of the image-correction process". Specifically, the process used to produce image 5-40 comprised omitting the captured image associated with imaging angle φ, and some of its neighbors, when the set of simulated images corresponding to imaging angle φ was generated. The image 5-40 has a fit of 0.98 with the ideal reconstruction result 5-10, as calculated over the area 5-50.

Those skilled in the art will realize that the inventive principle may be modified in various ways without departing from the scope of the present invention as defined by the appended claims.

## Claims

1. A method comprising performing the following steps on a programmed data-processing apparatus:
a) obtaining (2-02) a sequence of images captured (1-500) from a three-dimensional (1-400) object by a penetrating radiation imaging apparatus (1-100) over a scanning period, wherein each captured image is associated with a specific imaging angle (φ), and wherein the three-dimensional object is transformed relative to the imaging apparatus in an unknown manner during the scanning period;
b) applying an initial reconstruction (2-04) on the sequence of the captured images, thereby creating an initial reconstructed volumetric image (3-510);
for each of several imaging angles (φ):
c) generating a set of simulated images (3-530) corresponding to the imaging angle (φ), by repeatedly applying simulated projections (2-14) on the initial reconstructed volumetric image under a set of varied spatial transformation parameters (3-520) of at least a portion of the initial reconstructed volumetric image relative to the imaging apparatus;
d) within the set of the simulated images (3-530) corresponding to the imaging angle (φ), determining (2-16) an optimized set of spatial transformation parameters (3-540), wherein the optimized set of spatial transformation parameters, when used for the simulated projection in the preceding step, results in an improved fit with the captured image associated with the imaging angle (φ) compared with the simulated projection without spatial transformation parameters; and
e) applying a second reconstruction (2-24) on the sequence of captured images, wherein the second reconstruction is corrected by the optimized set of spatial transformation parameters, thereby creating a transformation-corrected reconstructed volumetric image (3-560).

2. The method according to claim 1, wherein the spatial transformation parameters omit shifts along the imaging angle (φ).

3. The method according to claim 1 or 2, further comprising omitting the captured image associated with imaging angle (φ) when the set of simulated images corresponding to imaging angle (φ) is generated.

4. The method according to claim 3, further comprising omitting less than 50% of neighbors of the omitted captured image.

5. The method according to any one of the preceding claims, wherein the determination (2-16) of the optimized set of spatial transformation parameters (3-540) comprises one or more of a gradient optimization, a simplex method, a genetic algorithm and a simulated annealing.

6. The method according to any one of the preceding claims, further comprising iteratively repeating steps c) through e), by substituting the transformation-corrected reconstructed volumetric image (3-560) after each execution of step e) for the initial reconstructed volumetric image (3-510) in step c).

7. The method according to any one of the preceding claims, wherein the act of determining the optimized set of spatial transformation parameters comprises filtering the spatial transformation parameters in one or more combinations, wherein each combination comprises spatial transformation parameters for multiple imaging angles.

8. A programmed data-processing apparatus comprising at least one processing unit (4-110), memory (4-150) for storing applications and data, wherein the memory comprises program code instructions (4-160) for instructing the at least one processing unit to carry out the following steps:
a) obtaining (2-02) a sequence of images captured (1-500) from a three-dimensional (1-400) object by a penetrating radiation imaging apparatus (1-100) over a scanning period, wherein each captured image is associated with a specific imaging angle (φ), and wherein the three-dimensional object is transformed relative to the imaging apparatus in an unknown manner during the scanning period;
b) applying an initial reconstruction (2-04) on the sequence of the captured images, thereby creating an initial reconstructed volumetric image (3-510);
for each of several imaging angles (φ):
c) generating a set of simulated images (3-530) corresponding to the imaging angle (φ), by repeatedly applying simulated projections (2-14) on the initial reconstructed volumetric image under a set of varied spatial transformation parameters (3-520) of at least a portion of the initial reconstructed volumetric image relative to the imaging apparatus;
d) within the set of the simulated images (3-530) corresponding to the imaging angle (φ), determining (2-16) an optimized set of spatial transformation parameters (3-540), wherein the optimized set of spatial transformation parameters, when used for the simulated projection in the preceding step, results in an improved fit with the captured image associated with the imaging angle (φ) compared with the simulated projection without spatial transformation parameters; and
e) applying a second reconstruction (2-24) on the sequence of captured images, wherein the second reconstruction is corrected by the optimized set of spatial transformation parameters for the imaging angle (φ), thereby creating a transformation-corrected reconstructed volumetric image (3-560).

9. A computer-readable memory comprising program code instructions for a programmable data-processing apparatus, wherein execution of the program code instructions causes the programmable data-processing apparatus to carry out the method of claim 1.

## Patentansprüche

1. Verfahren, das das Durchführen der folgenden Schritte an einer programmierten Datenverarbeitungsvorrichtung umfasst:
a) Erhalten (2-02) einer Sequenz von Bildern, die während einer Scanperiode mittels einer Durchdringungsstrahlungsbildgebungsvorrichtung (1-100) an einem dreidimensionalen (1-400) Objekt erfasst (1-500) werden, wobei jedes erfasste Bild mit einem spezifischen Bildgebungswinkel (ϕ) verknüpft ist und wobei das dreidimensionale Objekt relativ zur Bildgebungsvorrichtung auf eine unbekannte Weise während der Scanperiode umgewandelt wird;
b) Anwenden einer anfänglichen Rekonstruktion (2-04) auf die Sequenz von erfassten Bildern, dadurch Erstellen eines anfänglichen rekonstruierten volumetrischen Bildes (3-510);
für jeden von mehreren Bildgebungswinkeln (ϕ):
c) Erzeugen eines Satzes von simulierten Bildern (3-530), die dem Bildgebungswinkel (ϕ) entsprechen, durch wiederholtes Anwenden von simulierten Projektionen (2-14) auf das anfängliche rekonstruierte volumetrische Bild unter einem Satz von variierten räumlichen Umwandlungsparametern (3-520) von mindestens einem Abschnitt des anfänglichen rekonstruierten volumetrischen Bildes relativ zur Bildgebungsvorrichtung;
d) im Satz der simulierten Bilder (3-530), die dem Bildgebungswinkel (ϕ) entsprechen, Bestimmen (2-16) eines optimierten Satzes von räumlichen Umwandlungsparametern (3-540), wobei der optimierte Satz von räumlichen Umwandlungsparametern, wenn er für die simulierte Projektion im vorhergehenden Schritt verwendet wird, verglichen mit der simulierten Projektion ohne räumliche Umwandlungsparameter in einem verbesserten Passsitz beim erfassten Bild, das mit dem Bildgebungswinkel (ϕ) verknüpft ist, resultiert; und
e) Anwenden einer zweiten Rekonstruktion (2-24) auf die Sequenz von erfassten Bildern, wobei die zweite Rekonstruktion durch den optimierten Satz von räumlichen Umwandlungsparametern korrigiert wird, dadurch Erstellen eines umwandlungskorrigierten rekonstruierten volumetrischen Bildes (3-560).

2. Verfahren nach Anspruch 1, wobei die räumlichen Umwandlungsparameter Verschiebungen entlang des Bildgebungswinkels (cp) weglassen.

3. Verfahren nach Anspruch 1 oder 2, das ferner das Weglassen des erfassten Bildes, das mit dem Bildgebungswinkel (cp) verknüpft ist, umfasst, wenn der Satz von simulierten Bildern, die dem Bildgebungswinkel (cp) entsprechen, erzeugt wird.

4. Verfahren nach Anspruch 3, das ferner das Weglassen von weniger als 50 % von Nachbarn des weggelassenen erfassten Bildes umfasst.

5. Verfahren nach einem der vorhergehenden Ansprüche, wobei das Bestimmen (2-16) des optimierten Satzes von räumlichen Umwandlungsparametern (3-540) eines oder mehreres von einer Gradientenoptimierung, einem Simplexverfahren, einem genetischen Algorithmus und einem simulierten Abkühlen umfasst.

6. Verfahren nach einem der vorhergehenden Ansprüche, das ferner das iterative Wiederholen der Schritte c) bis e) durch Ersetzen des umwandlungskorrigierten rekonstruierten volumetrischen Bildes (3-560) nach jeder Ausführung von Schritt e) durch das anfängliche rekonstruierte volumetrische Bild (3-510) in Schritt c) umfasst.

7. Verfahren nach einem der vorhergehenden Ansprüche, wobei der Vorgang des Bestimmens des optimierten Satzes von räumlichen Umwandlungsparametern das Filtern der räumlichen Umwandlungsparameter in einer oder mehreren Kombinationen umfasst, wobei jede Kombination räumliche Umwandlungsparameter für mehrere Bildgebungswinkel umfasst.

8. Programmierte Datenverarbeitungsvorrichtung, die mindestens eine Verarbeitungseinheit (4-110), Speicher (4-150) zum Speichern von Anwendungen und Daten umfasst, wobei der Speicher Programmcodeanweisungen (4-160) zum Anweisen der mindestens einen Verarbeitungseinheit, die folgenden Schritte umzusetzen, umfasst:
a) Erhalten (2-02) einer Sequenz von Bildern, die während einer Scanperiode mittels einer Durchdringungsstrahlungsbildgebungsvorrichtung (1-100) an einem dreidimensionalen (1-400) Objekt erfasst (1-500) werden, wobei jedes erfasste Bild mit einem spezifischen Bildgebungswinkel (ϕ) verknüpft ist und wobei das dreidimensionale Objekt relativ zur Bildgebungsvorrichtung auf eine unbekannte Weise während der Scanperiode umgewandelt wird;
b) Anwenden einer anfänglichen Rekonstruktion (2-04) auf die Sequenz von erfassten Bildern, dadurch Erstellen eines anfänglichen rekonstruierten volumetrischen Bildes (3-510);
für jeden von mehreren Bildgebungswinkeln (ϕ):
c) Erzeugen eines Satzes von simulierten Bildern (3-530), die dem Bildgebungswinkel (ϕ) entsprechen, durch wiederholtes Anwenden von simulierten Projektionen (2-14) auf das anfängliche rekonstruierte volumetrische Bild unter einem Satz von variierten räumlichen Umwandlungsparametern (3-520) von mindestens einem Abschnitt des anfänglichen rekonstruierten volumetrischen Bildes relativ zur Bildgebungsvorrichtung;
d) im Satz der simulierten Bilder (3-530), die dem Bildgebungswinkel (ϕ) entsprechen, Bestimmen (2-16) eines optimierten Satzes von räumlichen Umwandlungsparametern (3-540), wobei der optimierte Satz von räumlichen Umwandlungsparametern, wenn er für die simulierte Projektion im vorhergehenden Schritt verwendet wird, verglichen mit der simulierten Projektion ohne räumliche Umwandlungsparameter in einem verbesserten Passsitz beim erfassten Bild, das mit dem Bildgebungswinkel (ϕ) verknüpft ist, resultiert; und
e) Anwenden einer zweiten Rekonstruktion (2-24) auf die Sequenz von erfassten Bildern, wobei die zweite Rekonstruktion durch den optimierten Satz von räumlichen Umwandlungsparametern für den Bildgebungswinkel (ϕ) korrigiert wird, dadurch Erstellen eines umwandlungskorrigierten rekonstruierten volumetrischen Bildes (3-560).

9. Computerlesbarer Speicher, der Programmcodeanweisungen für eine programmierbare Datenverarbeitungsvorrichtung umfasst, wobei die Ausführung der Programmcodeanweisungen die programmierbare Datenverarbeitungsvorrichtung veranlasst, das Verfahren nach Anspruch 1 umzusetzen.

## Revendications

1. Procédé qui comprend l'exécution des étapes suivantes sur un appareil de traitement de données programmé :
a) l'obtention (2-02) d'une séquence d'images capturées (1-500) à partir d'un objet en trois dimensions (1-400) à l'aide d'un appareil d'imagerie de pénétration par rayonnement (1-100) pendant une période de balayage, dans lequel chaque image capturée est associée à un angle d'imagerie spécifique (Φ), et dans lequel l'objet en trois dimensions est transformé par rapport à l'appareil d'imagerie d'une manière inconnue pendant la période de balayage ;
b) l'application d'une reconstruction initiale (2-04) sur la séquence d'images capturées, afin de créer une image volumétrique reconstruite initiale (3-510) ;
pour chacun des angles d'imagerie (Φ) :
c) la génération d'un ensemble d'images simulées (3-530) qui correspondent à l'angle d'imagerie (Φ), en appliquant de manière répétée des projections simulées (2-14) sur l'image volumétrique reconstruite initiale sous un ensemble de paramètres de transformation spatiale variés (3-520) d'au moins une partie de l'image volumétrique reconstruite initiale par rapport à l'appareil d'imagerie ;
d) au sein de l'ensemble d'images simulées (3-530) qui correspondent à l'angle d'imagerie (Φ), la détermination (2-16) d'un ensemble optimisé de paramètres de transformation spatiale (3-540), dans lequel l'ensemble optimisé de paramètres de transformation spatiale, lorsqu'il est utilisé pour la projection simulée à l'étape précédente, engendre une meilleure adaptation avec l'image capturée associée à l'angle d'imagerie (Φ) en comparaison avec la projection simulée sans paramètres de transformation spatiale ; et
e) l'application d'une seconde reconstruction (2-24) sur la séquence d'images capturées, dans lequel la seconde reconstruction est corrigée par l'ensemble optimisé de paramètres de transformation spatiale, afin de créer une image volumétrique reconstruite à transformation corrigée (3-560).

2. Procédé selon la revendication 1, dans lequel les paramètres de transformation spatiale omettent les décalages le long de l'angle d'imagerie (Φ).

3. Procédé selon la revendication 1 ou 2, qui comprend en outre l'omission de l'image capturée associée à l'angle d'imagerie (Φ) lorsque l'ensemble d'images simulées qui correspondent à l'angle d'imagerie (Φ) est généré.

4. Procédé selon la revendication 3, qui comprend en outre l'omission de moins de 50 % de l'environnement de l'image capturée omise.

5. Procédé selon l'une quelconque des revendications précédentes, dans lequel la détermination (2-16) de l'ensemble optimisé de paramètres de transformation spatiale (3-540) comprend un(e) ou plusieurs d'une optimisation de gradient, d'un procédé simplex, d'un algorithme génétique et d'un recuit simulé.

6. Procédé selon l'une quelconque des revendications précédentes, qui comprend en outre la répétition des étapes c) à e), en remplaçant l'image volumétrique reconstruite à transformation corrigée (3-560) après chaque exécution de l'étape e) par l'image volumétrique reconstruite initiale (3-510) de l'étape c).

7. Procédé selon l'une quelconque des revendications précédentes, dans lequel la détermination de l'ensemble optimisé de paramètres de transformation spatiale comprend le filtrage des paramètres de transformation spatiale en une ou plusieurs combinaison(s), dans lequel chaque combinaison comprend des paramètres de transformation spatiale pour plusieurs angles d'imagerie.

8. Appareil de traitement de données programmé qui comprend au moins une unité de traitement (4-110), et une mémoire (4-150) destinée à stocker des applications et des données, dans lequel la mémoire comprend des instructions de code de programme (4-160) destinées à demander à la au moins une unité de traitement d'exécuter les étapes suivantes :
a) l'obtention (2-02) d'une séquence d'images capturées (1-500) à partir d'un objet en trois dimensions (1-400) à l'aide d'un appareil d'imagerie de pénétration par rayonnement (1-100) pendant une période de balayage, dans lequel chaque image capturée est associée à un angle d'imagerie spécifique (Φ), et dans lequel l'objet en trois dimensions est transformé par rapport à l'appareil d'imagerie d'une manière inconnue pendant la période de balayage ;
b) l'application d'une reconstruction initiale (2-04) sur la séquence d'images capturées, afin de créer une image volumétrique reconstruite initiale (3-510) ;
pour chacun des angles d'imagerie (Φ) :
c) la génération d'un ensemble d'images simulées (3-530) qui correspondent à l'angle d'imagerie (Φ), en appliquant de manière répétée des projections simulées (2-14) sur l'image volumétrique reconstruite initiale sous un ensemble de paramètres de transformation spatiale variés (3-520) d'au moins une partie de l'image volumétrique reconstruite initiale par rapport à l'appareil d'imagerie ;
d) au sein de l'ensemble d'images simulées (3-530) qui correspondent à l'angle d'imagerie (Φ), la détermination (2-16) d'un ensemble optimisé de paramètres de transformation spatiale (3-540), dans lequel l'ensemble optimisé de paramètres de transformation spatiale, lorsqu'il est utilisé pour la projection simulée à l'étape précédente, engendre une meilleure adaptation avec l'image capturée associée à l'angle d'imagerie (Φ) en comparaison avec la projection simulée sans paramètres de transformation spatiale ; et
e) l'application d'une seconde reconstruction (2-24) sur la séquence d'images capturées, dans lequel la seconde reconstruction est corrigée par l'ensemble optimisé de paramètres de transformation spatiale pour l'angle d'imagerie (Φ), afin de créer une image volumétrique reconstruite à transformation corrigée (3-560).

9. Mémoire lisible par un ordinateur qui comprend des instructions de code de programme destinées à un appareil de traitement de données programmable, dans laquelle l'exécution des instructions de code de programme permet à l'appareil de traitement de données programmable d'exécuter le procédé selon la revendication 1.
